Europäisches Patentamt

European Patent Office

Office européen des brevets

(11) Publication number: **0 251 460 B1**

(19)

(12) **EUROPEAN PATENT SPECIFICATION**

(45) Date of publication of patent specification: **12.08.92**

(21) Application number: **87304314.5**

(22) Date of filing: **15.05.87**

(51) Int. Cl.⁵: **C12N 15/51**, C12N 1/16, A61K 39/29

(54) **Method for producing hepatitis B virus core antigen (HBcAg) in yeast.**

(30) Priority: **23.05.86 US 866558**

(43) Date of publication of application:
**07.01.88 Bulletin 88/01**

(45) Publication of the grant of the patent:
**12.08.92 Bulletin 92/33**

(84) Designated Contracting States:
**AT BE CH DE ES FR GB IT LI LU NL SE**

(56) References cited:
**EP-A- 0 103 201**
**EP-A- 0 174 444**

**CHEMICAL ABSTRACTS, vol. 102, no. 17, 29th April 1985, page 149, abstract no. 144119t, Columbus, Ohio, US; P. VALENZUELA et al.: "Synthesis and assembly of hepatitis B virus antigens in heterologous systems", & HEPATITIS B: VIRUS, DIS., VACCINE, [PROC. SYMP.] 1982 (Pub. 1984), 225-36**

(73) Proprietor: **MERCK & CO. INC.**
**126, East Lincoln Avenue P.O. Box 2000**
**Rahway New Jersey 07065-0900(US)**

(72) Inventor: **Ellis, Ronald W.**
**1407 Edgevale Road**
**Overbrook Hills Pennsylvania 19440(US)**
Inventor: **Hagopian, Arpi**
**771 Hartley Drive**
**Lansdale Pennsylvania 19446(US)**
Inventor: **Kniskern, Peter J.**
**841 Patterson Drive**
**Lansdale Pennsylvania 19446(US)**

(74) Representative: **Hesketh, Alan, Dr.**
**European Patent Department Merck & Co., Inc. Terlings Park Eastwick Road Harlow Essex, CM20 2OR(GB)**

GENE, vol. 32, no. 3, part 2, December 1984, pages 263-274, Elsevier Sience Publishers, Amsterdam, NL; G.A. BITTER et al.: "Expression of heterologous genes in Saccharomyces cerevisiae from vectors utilizing the glyceraldehyde-3-phosphate dehydrogenase gene promoter"

BIOTECHNOLOGY, vol. 3, April 1985, pages 317-320; P. VALENZUELA et al.: "Synthesis and assembly in yeast of hepatitis B surface antigen particles containing the polyalbumin receptor"

PROC. NATL. ACAD. SCI. USA, vol. 80, January 1983, pages 1-5; A. MIYANOHARA et al.: "Expression of hepatitis B surface antigen gene in yeast"

CHEMICAL ABSTRACTS, vol. 105, no. 5, August 1986, page 194, abstract no. 36817x, Columbus, Ohio, US; A. MIYANOHARA et al.: "Expression of hepatitis B virus core antigen gene in Saccharomyces cerevisiae: synthesis of two polypeptides translated from different initiation codons", & J. VIROL. 1986, 59(1), 176-80

GENE, vol. 46, no. 1, 1986, pages 135-141, Elsevier Science Publishers B.V. (Biomedical Division), Amsterdam, NL; P.J. KNISKERN et al.: "Unusually high-level expression of a foreign gene (hepatitis B virus core antigen) in Saccharomyces cerevisiae "

## Description

BACKGROUND OF THE INVENTION

Hepatitis B virus (HBV) is an infectious agent responsible for several human liver diseases, with a high incidence of infection occurring perinatally from chronically-infected mothers to their children. Most individuals infected by HBV suffer through an acute phase of disease followed by recovery, but a large number of individuals fail to clear their infection. The number of chronic carriers worldwide has been estimated at over two hundred million, and hundreds of thousands die annually from the long-term consequences of chronic hepatitis B (cirrhosis or hepatocellular carcinoma).

Of the two types of structural proteins in the hepatitis B (HB) virion, the envelope of surface ("S") protein and the core ("C") protein, the core ("C") protein is the structural protein of the virion nucleocapsid. This "C" protein (HBcAg) is the product of an open reading frame (ORF) of 636 nucleotides amino acids and is expressed as intracellular particles as well as on the membranes of infected hepatocytes. The "C" protein's ORF is demarcated into two regions, each of which begins with an ATG codon which is capable of translational initiation in vivo. The presence in virion genomic DNA of a nick at base 8 downstream from the first ATG makes initiation of transcription from the first ATG an improbable event and results in the major synthesized product found in vivo being that initiated from the second ATG. These domains are referred to as Pre-C (87 nucleotides) and C (549 nucleotides) in their 5′--3′ order in the ORF. The product of the C gene (183 amino acids) encompasses all the major epitopes of both the nucleocapsid and core particle.

The ability of "S" antigens (HBsAg) to induce the production of protective antibodies (anti-HBs) in human recipients is well documented [New England J. Med., 303, 833-841 (1980) and 307, 481-1486 (1982); and JAMA, 251, 2812-2815 (1984)]. However, in some cases, and for some patient groups, vaccination with "S" antigens does not elicit protective titers of anti-HBs [Lancet, 1, 346-347 (1984); Clin. Microbiol., 18, 305-309 (1983); and New England J. Med., 311, 496-499 (1984)]. In addition, cell-mediated immunity (CMI) to HBsAg has not been demonstrated.

However, both humoral and CMI responses to HBcAg have been obtained in laboratory animals. In fact, recent protective efficacy studies in chimpanzees have demonstrated amelioration of infection in animals immunized with plasma-derived or E coli-derived HBcAg preparations [Gastroenterology, 88, 763-7 (1985)]. But antibodies to HBcAg (anti-HBc) co-exist with an infectious state and transplacentally-acquired anti-HBc has been shown to provide no protection for HBV-exposed neonates. On the other hand, vaccine which could be shown to be capable of eliciting a CMI response to HBcAg would therefore be expected to facilitate the destruction of infected hepatocytes by cytotoxic T-lymphocytes.

During the course of an HBV infection, the appearance of anti-HBc is a sensitive and lonasting marker of infection and of the chronic carrier state. Thus, a means of detecting anti-HBc is a valuable screen for human blood and blood products. To this end, a number of manufacturers have developed anti-HBc tests which employ HBcAg purified from infectious plasma as the source of test reagent (e.g., CorAb[R] Test, Abbott Labs). The availability of an alternative source of HBcAg with immunologic identity to the plasma-derived product and which is free of other HBV antigens would be expected to provide an economic, specific, and non-infectious source of antigen for such immunodiagnostics.

EP-A-0 103 201 discloses the preparation of HBcAg gene expression plasmid. There is no suggestion of introduction of a yeast transscriptional termination sequence.

Chemical Abstracts, vol. 102, no.17, April 1985, page 149, abstr. no. 144119t discloses expression of an HBcAg in E. coli transformants containing the HbcAg gene fused to the lac operon promoter. No mention is made of a yeast transscriptional termination sequence.

In order to expand the available supply of HBcAg for vaccines and immunodiagnostics, we sought to express high levels of HBcAg by recombinant DNA technology in microbial cells. Yeast has shown the ability to express immunologically active HBsAg particles which have been as effective in human clinical trials, as plasma-derived HBsAg [JAMA, 251, 2812-2815 (1984)]. For this reason, yeast was chosen to serve as the host for the expression of recombinant HBcAg in an immunologically-active form. In addition, expression of human therapeutic agents and vaccines in yeast can be very useful for product development, since yeast is free of endotoxin, is nonpathogenic to man, can be fermented to industrial scale, and lacks many of the safety concerns which surround the use of continuous mammalian cell lines (many of which are virally transformed, may be tumorigenic in mice and all of which contain protooncogenes).

It was, therefore, an object of the present invention to provide expression vectors and processes for the expression of HBcAg in yeast as an immunogenic particle. Another object of this invention was to specify conditions for the scale-up of the growth of recombinant host cells transformed by such vectors, such that maximal yields of HBcAg may be attained in much larger volumes and in higher concentrations for the purification of such polypeptides. These and other objects of the present invention will be apparent from the following description.

## BRIEF DESCRIPTION OF THE DRAWINGS

Figure 1 - Preparation procedure of Y5'Y3' HBcAg expression plasmid.

Figure 2 - Electrophorogram and electron micrographs of HBcAg in S. cerevisiae.

## SUMMARY OF THE INVENTION

The HBcAg gene has been expressed in yeast. The expressed protein aggregates into a particulate form which displays the major antigenic sites encoded by plasma-derived HBcAg, thereby highlighting the utility of yeast as a host for the expression of this gene. This protein is useful for in vitro diagnostic systems and as a vaccine for the prevention of HBV-induced infections.

## DETAILED DESCRIPTION OF THE INVENTION

The present invention is directed to a method for the expression of HBcAg in yeast species.

Dane particles are utilized as the source of HBV nucleic acid (for the isolation of the HBcAg ORF). The endogenous polymerase reaction is employed in order to produce covalently-closed circular double-stranded DNA (repaired) of the HBV genome from the nicked and gapped nucleic acid form that natively resides in the HB virion. The repaired DNA is isolated, digested to completion with the restriction enzyme, EcoRI, and cloned into the EcoRI site of plasmid, pBR322. The recombinant plasmids thus produced are selected, these containing the HBV genome in a circularly permuted form at the EcoRI site of the Pre-S region. The HBcAg gene is isolated from such recombinant pBR322 by digestion with the restriction enzymes, HhaI and AvaI, isolation of the 0.6 kilobase-pair (kbp) fragment, and digestion with the enzyme, TaqI, to yield a 0.5 kbp DNA fragment isolated by preparative agarose gel electrophoresis with TaqI and AvaI termini. The missing bases at the 5' and 3' ends of the HBcAg are reconstituted to the original composition with the appropriate synthetic oligonucleotides, yielding a 0.7 kbp fragment with the restriction enzyme, HindIII termini containing an optimized nontranslated leader (NTL) sequence and the complete HBcAg ORF.

The plasmid pGAP-tADH-2 contains a unique HindIII site [between the glyceraldehyde phosphate dehydrogenate (GAP) promoter and the alcohol dehydrogenase (ADH) transcriptional terminator] into which the complete ORF described above is inserted in the appropriate orientation. This 2.5 kbp expression cassette then is removed by SphI digestion and ligated into the shuttle vector pC1/1 at the SphI site. This vector is used to transform a yeast strain and transformed clones thus produced are selected. Recombinant yeast are grown in synthetic leu⁻ minimal medium. After growth to stationary phase, yeast cells are harvested, and lysates are prepared and resolved by sodium dodecylsulfate polyacrylamide electrophoresis (SDS-PAGE). A major polypeptide is found with a monomeric molecular weight of 22 (kD) kilodaltons in accord with the predicted molecular weight of the translational product of the HBcAg ORF. Furthermore, lysates of recombinant, but not parental yeast, are always positive for HBcAg by radioimmunoassay. Electron microscopic examination of fresh yeast lysates shows high densities of typical 28nm HBcAg particles.

The yeast-derived promoter initiates transcription of the HBcAg gene. Therefore, it is obvious to those skilled in the art that any yeast promoter may be substituted for the GAP promoter. It is also obvious to those skilled in the art that a suitable assay system, e.g., Western blot or radioimmunoassay or enzyme-linked immunoassay (EIA), should be utilized in order to assay expression of HBcAg polypeptides in this system, such that the time of harvesting of the culture for attaining a maximal yield can be optimized.

The genus Saccharomyces is composed of a variety of species. The most commonly used is Saccharomyces cerevisiae, or baker's yeast, as a host for the recombinant DNA-mediated expression of a variety of foreign polypeptides. However, the distinctions among other species of the genus Saccharomyces are not always well-defined. Many of these species are capable of interbreeding with S. cerevisiae and are likely to possess promoters which are analogous to or identical to promoters in S. cerevisiae. Therefore, it will be obvious to those skilled in the art that, for the expression of HBcAg polypeptides, the selection of a host strain extends to other species of the genus Saccharomyces, including but not limited to carlsbergensis, uvarum, rouxii, montanus, kluyveri, elongisporus, norbensis, oviformis, and diastaticus.

Several yeast genera, such as Hansenula, Candida, Torulopsis, and Pichia, have been shown to contain similar metabolic pathways for the utilization of methanol as a sole carbon source for growth. The gene for alcohol oxidase, an enzyme which participates in this metabolic pathway, has been isolated from Pichia pastoris. The P. pastoris alcohol oxidase promoter has been isolated and shown to be susceptible to methanol induction of expression. Such an inducible promoter is useful for the expression of polypeptides in yeast. In particular, this promoter has been shown to be active on a plasmid for the inducible expression of the HBV "S" domain in P. pastoris in particulate form. This observation highlights the ability of other yeast genera to function as hosts for the recombinant DNA-mediated expression of polypeptides in immunologically-active form. Therefore, it will be obvious to those skilled in the art that, for the expression of HBcAg, the selection of a host strain extends to species from other genera of yeast from the Families Saccharomycetaceae and Cryptococcaceae, including, but not limited to Pichia, Candida, Hansenula, Torulopsis, Kluyveromyces, and Saccharomycopsis.

The following examples illustrate the present invention without, however, limiting the same thereto. The disclosure of each reference mentioned in the following examples is hereby incorporated by reference.

EXAMPLE I

Cloning of HBV DNA in pBR322

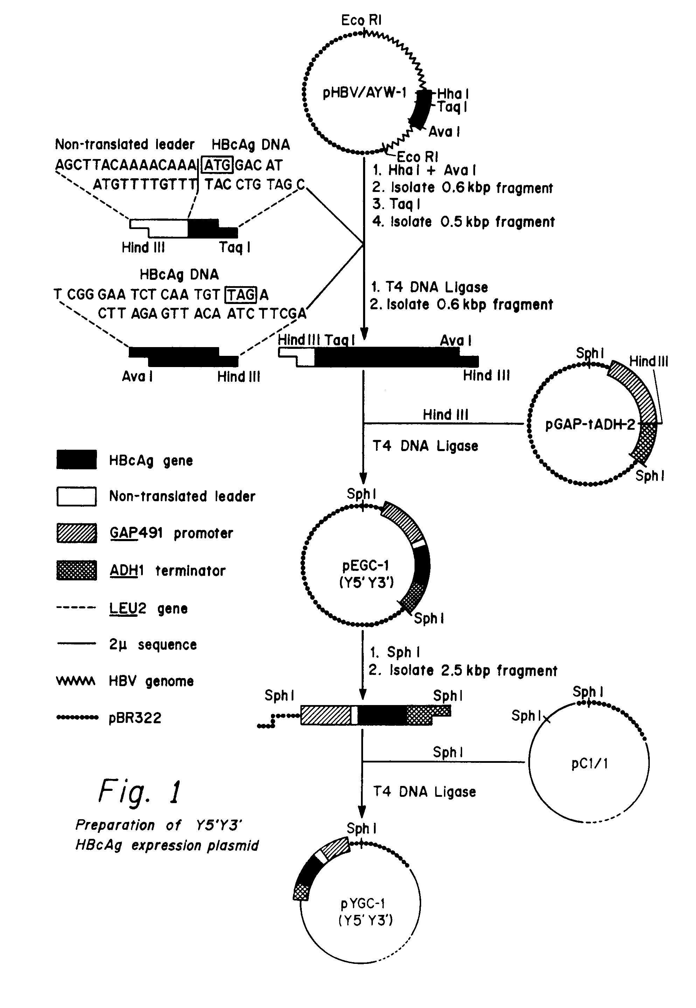

HBV Dane particles were isolated and purified from human plasma (carrier), and double-stranded DNA was synthesized by endogenous polymerase in the Dane particles according to the methods of Landers, et al, ["J. Virology" 23, 268 (1977)] and Hruska et al, [J. Virology, 21, (1977)]. The DNA was isolated after digestion with Proteinase K in SDS followed by extraction with phenol/chloroform and ethanol precipitation. The HBV genomic DNA was digested with EcoRI, producing a single 3.2 kbp fragment, and cloned into the EcoRI site of pBR322. The presence of the HBV DNA was confirmed by EcoRI digestion, Southern blot transfer to nitrocellulose, and hybridization with [$^{32}$P]-labelled specific oligonucleotide probes. This plasmid is referred to as HBV/AYW-1 (Figure 1).

EXAMPLE II

Cloning of the HBcAg Gene into the pGAP-tADH-2 Expression Vector

As shown in Figure 1, plasmid HBV/AYW-1 (described in Example I) was digested with HhaI and AvaI and the 0.6 kbp fragment purified by preparative agarose gel electrophoresis. After subsequent digestion with TaqI, the resulting 0.5 kbp fragment also was purified by preparative agarose gel electrophoresis to yield a fragment with TaqI and AvaI termini.

To reconstruct the 5' portion of the HBcAg ORF, a pair of oligonucleotides was synthesized which reconstitutes the ORF from the TaqI site upstream to the ATG through a 10bp NTL sequence to a HindIII terminus. The sequence of this oligonucleotide is:

**AGCTTACAAAACAAAATGGACAT**

**ATGTTTTGTTTTACCTGTAGC**

To reconstitute the 3' portion of the HBcAg ORF, a second pair of oligonucleotides was synthesized which reconstitutes the ORF from the AvaI site through the translational terminator to a HindIII terminus. The sequence of this oligonucleotide is:

**TCGGGAATCTCAATGTTAGA**

**CTTAGAGTTACAATCTTCGA**

The plasmid pGAP-tADH-2 (see Fig. 1) containing the GAP491 promoter [Holland & Holland, J. Biol. Chem. 255: 2596 (1980)] and the ADH1 transcriptional terminator in pBR322, has a unique HindIII cloning site into which the HBcAg ORF described above was ligated, yielding pEGC-1 (Figure 1). The presence and orientation of HBcAg DNA was confirmed by restriction endonuclease analyses and Southern blot transfer. The expression cassette containing the HBcAg ORF was removed from pEGC-1 by SphI digestion and isolated by preparative agarose gel electrophoresis. The cassette was then cloned into the shuttle vector pC1/1 [Beggs, Nature 275: 104 (1978); Rosenberg et al., Nature, 312: 77 (1984)] which had been digested previously with SphI. The resultant plasmid containing the expression cassette (pYGC-1) was used to transform S. cerevisiae strain 2150-2-3 [MATa, adel, leu204°, obtained from Dr. Pablo Valenzuela, Chiron Corporation, Emeryville, CA, which was derived from a genetic cross between strain Y379-5-D cyh2 nibl (rho⁻) Livingston, D. Genetics 86, 73 (1977) and DC 04 a Adel AdeX leu2-04 (cir°) (Broach, J., Cell, 21, 501 (1980)]. The transformation was effected following the procedure of Hinnen et al., Proc. Natl. Acad. Sci. USA 75 (1978) 1929-1933 and clones were selected for evaluation as described in Example III.

EXAMPLE III

Growth and Expression of HBcAg Gene

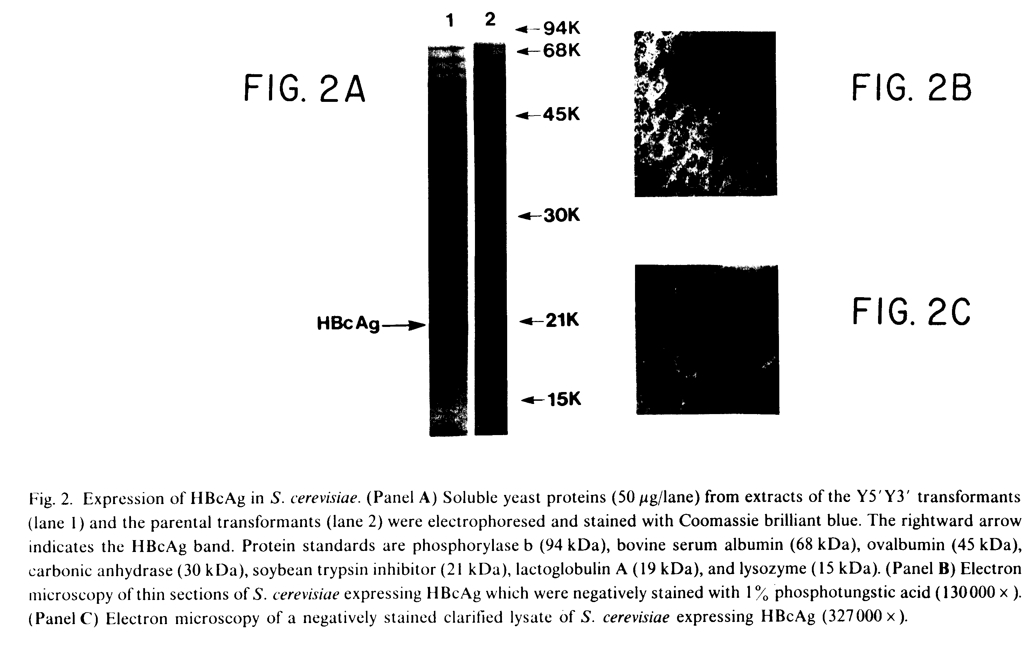

Clones of yeast containing the expression plasmid described in Example II were suspended in 0.2-0.3 ml $H_2O$, plated into SD-leucine selective agar plates, and incubated at 30° for 2-3 days. These yeast were inoculated into 5-7 ml cultures of leucine⁻ selective media [Sherman et al, "Methods in Yeast Genetics" (1983), Cold Spring Harbor Laboratory, N.Y.], and the cultures were incubated at 30° with aeration for 12-18 hours. Flasks containing 50 ml selective media were inoculated from the above cultures at a dilution 1:25 and were incubated at 30°C with shaking (350 rpm) for 48-72 hours to a final $A^{600}$ of 2.8-3.2. Duplicate samples of 5 $A^{600}$ units were aliquoted into tubes, and the yeast cells were pelleted at 2000xg for 10 min. The pellets were resuspended in 0.3 ml of phosphate-buffered saline containing 2mM phenylmethyl sulfonyl fluoride and transferred to 1.5 ml eppendorf tubes. Yeast cells were broken by the addition of 200-300 mg of washed glass beads (0.45mm) and agitation on a vortex mixer for 5-15 min. Cellular debris and glass beads were removed by centrifugation at 2000xg for 10 min. The clarified supernatant fluid was removed and assayed for protein [by the method of Lowry et al, J. Biol. Chem. 193, 265 (1951)] and HBcAg radioimmune assay specific for HBcAg. Typical values for antigen expression were 200-500 mg HBcAg/ml of yeast culture and 0.3-0.45 mg HBcAg/mg soluble yeast protein. Levels of expression were estimated to be 20-30% of soluble yeast proteins by Coomassie-blue SDS-PAGE stained with Coomassie Brilliant Blue (Figure 2). Electron micrographs (Figure 3) of lysed yeast cells clearly demonstrated that the HBcAg assembled into 28 nm particles which were indistinguishable from naturally occurring HBcAg particles (isolated from hepatic tissue or virions) (Figure 3).

EXAMPLE IV

Large Scale Growth and Purification of HBcAg

Recombinant S. cerevisiae, constructed as described in Example II, were grown in a 16 liter New Brunswick Scientific Fermenter charged with 9.5 liters of leucine⁻/synthetic selective media. Fermentation conditions were 500 rpm agitation, 5 liters air per min. at 30°C for 40 hours to a final $A^{660} = 1.6$. The yeast cells were harvested by microfiltration in an Amicon DC-10 unit, (with a H5M POI cartridge (with a molecular weight cutoff of $10^6$ daltons), suspended in 30 ml. 0.1M $Na_2HPO_4$, pH 7.1, 0.5M NaCl, and broken in a Stansted pressure cell for 7 passages at 75-85 pounds per square inch. The broken suspension was clarified by centrifugation at 10,000xg for 20 min. at 4°C. The clarified broth was processed to purify HBcAg particles in a manner described previously [Murray et al, EMBO J. 3: 645 (1984)].

Claims

1. A plasmid expression vector containing yeast-derived sequences for the selection and amplification of the plasmid in a species of yeast selected from the families Saccharomycetaceae or Cryptococcaceae, a yeast promoter, the HBcAg coding region, and a yeast transcriptional termination sequence.

2. A plasmid expression vector according to Claim 1 wherein the yeast promoter is from the glyceraldehyde phosphate dehydrogenase gene.

3. A species of yeast selected from the families Saccharomycetaceae or Crytococcaceae containing a plasmid of Claim 1.

4. A species of yeast according to Claim 3 wherein the species is from the genus Saccharomyces.

5. A species of yeast according to Claim 4 wherein the species is Saccharomyces cerevisiae or Saccharomyces carlsbergensis.

6. A process for obtaining HBcAg of Claim 1 comprising:
   a. transforming cells from a strain selected from the families Saccharomycetaceae or Cryptococcaceae with a plasmid expression vector of Claim 1,
   b. culturing the transformed cells, and
   c. recovering the peptide from the resulting cultured cells or growth medium.

7. A process according to Claim 6 wherein the yeast is from the genus Saccharomyces from the family Saccharomycetaceae.

8. A process according to Claim 7 wherein the yeast is the species Saccharomyces cerevisiae or Saccharmyces carlsbergensis.

Revendications

1. Vecteur d'expression plasmidique, contenant des séquences dérivées de levure pour la sélection et l'amplification du plasmide dans une espèce de levure choisie parmi les familles Saccharomycetaceae ou Cryptococcaceae, un promoteur de levure, la région codant pour l'antigène HBcAg, et une séquence de terminaison de transcription de levure.

2. Vecteur d'expression plasmidique selon la revendication 1, dans lequel le promoteur de levure est celui du gène de la glycéraldéhyde phosphate déshydrogénase.

3. Espèce de levure choisie parmi les familles Saccharomycetaceae ou Cryptococcaceae, contenant un plasmide selon la revendication 1.

4. Espèce de levure selon la revendication 3, appartenant au genre Saccharomyces.

5. Espèce de levure selon la revendication 4, qui est Saccharomyces cerevisiae ou Saccharomyces carlsbergensis.

6. Procédé d'obtention de l'antigène HBcAg selon la revendication 1, dans lequel :
   a. on transforme des cellules d'une souche choisie parmi les familles Saccharomycetaceae ou Cryptococcaceae, avec un vecteur d'expression plasmidique selon la revendication 1 ;
   b. on cultive les cellules transformées ; et
   c. on récupère le peptide à partir des cellules cultivées résultantes ou du milieu de culture.

7. Procédé selon la revendication 6, dans lequel la levure est du genre Saccharomyces appartenant à la famille Saccharomycetaceae.

8. Procédé selon la revendication 7, dans lequel la levure appartient à l'espèce Saccharomyces cerevisiae ou Saccharomyces carlsbergensis.

**Patentansprüche**

1. Plasmidexpressionsvektor, welcher von Hefe abgeleitete Sequenzen zur Selektion und Verstärkung des Plasmids in einer Hefespezies, ausgewählt aus den Familien Saccharomycetaceae oder Cryptococcaceae, einen Hefe-Promotor, die HBcAg-Kodierungsregion sowie eine Hefe-Transkriptionsterminierungssequenz enthält.

2. Plasmidexpressionsvektor nach Anspruch 1, worin der Hefepromotor aus dem Glyceraldehydphosphatdehydrogenasegen stammt.

3. Hefespezies, ausgewählt aus den Familien Saccharomycetaceae oder Cryptococcaceae, welche ein Plasmid nach Anspruch 1 enthält.

4. Hefespezies nach Anspruch 3, worin die Spezies aus der Gattung Saccharomyces stammt.

5. Hefespezies nach Anspruch 4, worin die Spezies Saccharomyces cerevisiae oder Saccharomyces carlsbergensis ist.

6. Verfahren zur Erzeugung von HBcAg nach Anspruch 1 durch:
   a. Transformieren von Zellen aus einem aus den Familien Saccharomycetaceae oder Cryptococcaceae ausgewählten Stamm mit einem Plasmidexpressionsvektor nach Anspruch 1,
   b. Kultivieren der transformierten Zellen und
   c. Gewinnen des Peptids aus den resultierenden kultivierten Zellen oder dem Wachstumsmedium.

7. Verfahren nach Anspruch 6, worin die Hefe aus der Gattung Saccharomyces der Familie Saccharomycetaceae stammt.

8. Verfahren nach Anspruch 7, worin die Hefe die Spezies Saccharomyces cerevisiae oder Saccharomyces carlsbergensis ist.

EP 0 251 460 B1

Fig. 1

Preparation of Y5'Y3'
HBcAg expression plasmid

Fig. 2. Expression of HBcAg in *S. cerevisiae*. (Panel A) Soluble yeast proteins (50 µg/lane) from extracts of the Y5′Y3′ transformants (lane 1) and the parental transformants (lane 2) were electrophoresed and stained with Coomassie brilliant blue. The rightward arrow indicates the HBcAg band. Protein standards are phosphorylase b (94 kDa), bovine serum albumin (68 kDa), ovalbumin (45 kDa), carbonic anhydrase (30 kDa), soybean trypsin inhibitor (21 kDa), lactoglobulin A (19 kDa), and lysozyme (15 kDa). (Panel B) Electron microscopy of thin sections of *S. cerevisiae* expressing HBcAg which were negatively stained with 1% phosphotungstic acid (130 000 × ). (Panel C) Electron microscopy of a negatively stained clarified lysate of *S. cerevisiae* expressing HBcAg (327 000 × ).

EP 0 251 460 B1